# EUROPEAN PATENT SPECIFICATION

(11) **EP 2 745 818 B1**
(45) Date of publication and mention of the grant of the patent: **06.01.2021**
(21) Application number: 13466036.4
(22) Date of filing: 12.12.2013
(51) Int. Cl.: A61F 9/00, A61M 3/02, A61F 9/007, A61F 13/15, A61F 13/36

(54) **Device for removal of undesirable substances**
Gerät zum Entfernen von unerwünschten Substanzen
Appareil pour le retrait de substances indésirables

(30) Priority: 19.12.2012 CZ 20120922
(43) Date of publication of application: 25.06.2014
(73) Proprietor: Palasová, Lucie, 165 00 Praha 6 (CZ)
(72) Inventor: Palasová, Lucie, 165 00 Praha 6 (CZ)
(74) Representative: Pavlica, Tomas

(56) References cited:
- CN-A- 101 065 426
- GB-A- 1 060 288
- GB-A- 1 217 910
- GB-A- 1 332 281
- US-A1- 2005 085 759
- US-A1- 2009 216 172
- US-A1- 2011 106 121

## Description

### Field of the Invention

The invention concerns a device for removal of undesirable substances and items, for instance, organic waste products from the eye corners (rheums), further for removal of dust, sand grains, insects, fibres, dead cells and pollen that is applicable to adults, children or animals, with a low risk of injury to the eye or infection of the eyes.

The solution is especially suitable for removal of cosmetics residues, mascara particles, liquid liners and the like, from the eye areas with make, without disrupting the make-up or smearing of the make-up around the eyes.

### Background of the Invention

Removal of undesirable items or substances from the eyes in medicine mainly by rinsing with physiologically pure water solutions (irrigation), often combined with extraction. Apart from the procedures with usual resources, such as syringes, rinsing bottles and the like, a lot of equipment was designed for this task as described, for instance, in American patents - US Patent No. 6,261,275, 6,602071 and 6,969,368. But these devices are mostly impractical for use in ordinary life because the excess fluid can remove the cosmetics from around the eyes, soil clothes, etc.

A device according to the preamble of claim 1 is known from the document US-A-2005/085759.

In ordinary life, people solve the frequent problem of removal of rheums, dust, impurities, insects and cosmetics residues from around the eyes by improvisation, such as removal or scraping off with the fingers, nails, ear cotton sticks, handkerchief corners or rinse them with eye drops. Improvised devices may be contaminated, add more dirt to the eye (e.g. fibres, fragments, etc.), irritate or even damage the sensitive tissue of the eyes.

Fingers and nails are often unwashed and may irritate the eye as well as transfer infection to the eyes (infection originators: viruses, bacteria, parasites or fungi). This may subsequently even result in serious health problems, such as conjunctivitis, damage to the epithelium of the cornea or its ulceration, creation of styes and other problems, which may require medical treatment. A further disadvantage of this solution is that if make-up is applied to the area around the eyes, then the make-up is smeared, which is a highly undesirable result!

Ear cotton swabs are made of cotton, which most often leaves cotton fibres around the eyes and also irritate the area around the eyes. They stick to the eyelashes and may cause blurred vision. A further disadvantage of this solution is that after usage of such dry swabs, the treated area most often itches and this may result in undesirable lacrimation and, hence, in turn also cause smearing of make-up around the eyes.

Eyedrops are mostly safe (if not contaminated or expired) and often used as a solution in households, if they are available in case of need. However, eyedrops are not so good for usage around the eyes, if make-up has been applied and we do not want to smear the make-up. There is a very high probability that eyedrops will run from around the canthus or other place, which is made-up and the entire make-up shall be disrupted and in some cases, this may be at very high financial cost.

The most common and relatively frequent injury to the eye is just transfer of a foreign body to the eye. The foreign body is most often found at the upper or lower eyelid, eventually in the canthus, from where it can be removed. In the attempt at its removal using an unsuitable method, the dirt can be pushed deeper, eventually cut into the tissue, from where it is no longer possible to remove it by improvisation. Even more complicated may be removal of dirt in the case of people that wear contact lenses. Often, mascara particles collect around the eyes or canthus and colour the canthus. In this way, they disrupt the overall appearance of the make-up. Very often, it happens in these situations that the person that is wearing make-up around the eyes cannot use the cited solution, e.g. eyedrops and needs quick aid to stop irritation by foreign bodies or cosmetics particles around the eyes, which negatively affect appearance.

The cited deficiencies to a substantial degree are removed by the device of substances and items from around the eyes according to the invention.

### Summary of the Invention

The subject of the invention is a device for removal of undesirable substances and items, such as metabolic waste products, soiling, dust and cosmetics residues from the eye area, whose substance lies in that it comprises a functional part from porous hydrophilic polymer, which is shaped for non-aggressive contact with the cleaned area of the eye and comprises in its porousness a non-toxic and non-irritant fluid; a handle permanently bonded to or separable from the functional part; and a watertight packaging, in which the functional part and optionally the handle are sealed. Conveniently, device has a porous polymer functional part of average porosity 20 to 98 % of volume in relation to the total volume of the porous polymer. Conveniently, the porous polymer has a functional part of average pore size 0.1 to 250 microns. Conveniently, the porous polymer is a functional part of the deformable porous polymer adapted for extrusion of the captured non-toxic and non-irritant liquid by deformation of the operative part. There is also described a mode of production of the device defined above, whose substance lies in that the functional part is obtained from the water solution of the porous polymer precursor by extrusion of the porous polymer by removal of water from the stated solution, by which the acquired functional part eventually bonds with the handle and is eventually sealed in watertight packaging, separately or with the handle. Conveniently, the water solution of the porous polymer precursor is a water solution of polyvinyl alcohol and the porous polymer is exuded from this solution by separation of the water by means of recurrent freezing and thawing of the given solution.

There is also described a further mode of production of the device defined above, whose substance lies in that the water solution of the porous polymer precursor is a water solution that contains the majority part of 2-hydroxyethyl methacrylate and minority part of the multifunctional cross-linker and extrusion of the porous polymer is done by radical polymerisation in the presence of a water soluble initiator of radical polymerisation, by which the acquired functional part eventually bonds with the handle and is eventually sealed in watertight packaging, separately or with the handle.

Conveniently, the expulsion of the porous polymer from the water solution of the porous polymer precursor is done directly in the watertight packaging.

Conveniently, the non-toxic and non-irritant liquid is at least one fluid selected from a set including water, glycerol, 1,2-propylene glycol, triethyleneglycol, dimethyl sulfoxide, glycerol formal and sodium chloride. Conveniently, the non-toxic and non-irritant liquid is a water isotonic solution suitable for ophthalmic application. Conveniently, the porous polymer is a hydrophilic polymer, whose contact angle with the non-toxic and non-irritant fluid is less than 90°, more conveniently less than 75°. Conveniently, the porous polymer has pores that basically have Gaussian distribution.

Conveniently, the size of the pores grows from inside outwards toward the surface of the functional part. Conveniently, positive capillary pressure is established in the pores of the porous polymer upon contact with the non-toxic and non-irritant fluid. Conveniently, the porous polymer is selected from a set including polyurethane, polyurea, polyvinyl alcohol and its derivatives and the derivatives of polyacrylate and polymethacrylate acid. Conveniently, the porous polymer has covalent cross-linkage. Conveniently, the derivatives of polyacrylate and polymethacrylate acid include amides, nitriles and esters with polyols. Conveniently, the given esters are created by 2-hydroxyethyl methacrylate. Conveniently, the functional part has a rounded applicator end for non-aggressive contact with the area around the eye. Conveniently, the functional part has a cylindrical shape of tapering diameter 3 - 8 mm, more conveniently 4 - 6 mm, which at the end conically passes into the rounded applicator end of diameter 0.5 mm - 3 mm, more conveniently 1 - 2 mm. Conveniently, the non-toxic and non-irritant fluid at the instant of non-aggressive contact with the area around the eye is contained in a volume of 20 to 95 %, more conveniently 33 to 67 % of the maximum retention capacity of the functional porous polymer part. Conveniently, the packaging is closed with a watertight cap that is at one end linked to the functional part and at the other end to the handle.

### Brief Description of the Drawings

On the attached drawings:
Figure 1 shows the schematic example of the design of the device, containing the functional part 1 with applicator end 1A and handle 2;
Figures 2A to 2D show examples of the design of the device in longitudinal and transverse section for various cross-sections of functional part 1 and handle 2, while the marking of these parts is identical to than in Figure 1;
Figure 3 shows the longitudinal and transversal section of the convenient shape of the functional part 1 of the device with rounded applicator end 1A and conically tapering cylindrical functional part (1), encircling the handle (2);
Figure 4 shows the convenient design of the device with its functional part 1 sealed in watertight packaging 3, sealed with a watertight cap 4, with waterproof passage through the handle 2, while the handle 2 and lid 4 make up a convenient single integral part from same material;
Figure 5 shows an example of the device design with disposable functional part 1 and handle 2 for repeated use, whereas the functional part 1 is closed with a watertight cap 4 in the packaging 3, whereas the cap is integrated with the inner part 2A of the handle 2 and has a nut part 5A and the handle 2 has the corresponding base part 5B; and
Figure 6 shows an example design of the device consisting of only the functional part with rounded applicator member 1A.

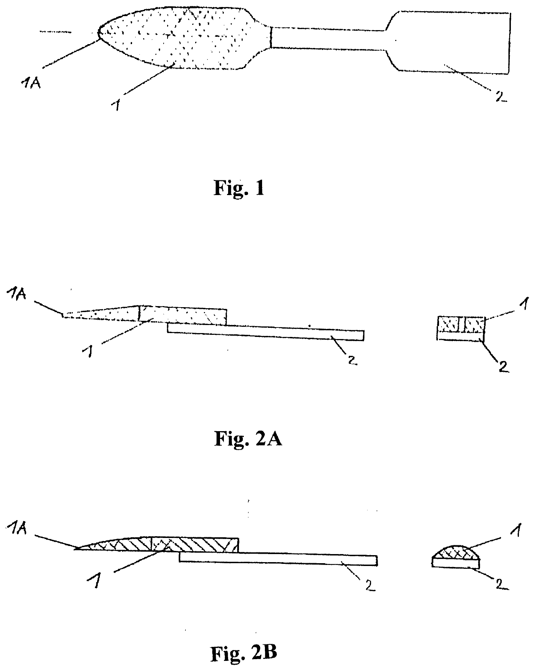

The device at least consists of the base functional part 1, schematically represented in Figure 1.

The functional part 1 that consists of the shaped soft porous part from hydrophilic polymer, which is prior to application in the area around the eye is at least partly soaked with a physiologically unobjectionable and non-irritant fluid. The applicator end 1A is intended for direct contact with the surface of the eye or its surroundings. The device further conveniently includes a handle 2 that is used to introduce the device to the area around the eye and its manipulation. Handle 2 is at the same time the carrier of functional part 1 and conveniently forms a single integral part with it. Handle 2 is usually made of suitable plastic material, but may also include other materials, such as metals or wood.

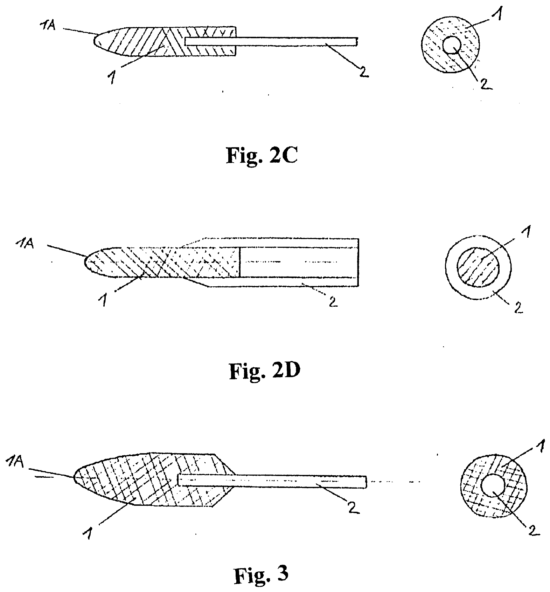

Porous functional part 1 has a shape optimised for contact with the selected area around the eye, e.g. canthus or eyelid. It may also have, for instance, a rectangular, semi-circular, oval or circular section. In the case of the rectangular or semi-circular profile, the functional part 1 is connected to the handle 2 on the flat side, as shown in Figure 2A and Figure 2B. In the case of the oval or circular profile, the handle 2 may be positioned either inside functional part 1, as shown in Figure 2C, or the functional part 1 may be positioned inside the handle 2, as shown in Figure 2D.

The functional part 1 and the handle 2 may be permanently bonded in various ways, e.g. gluing, welding, casting of the functional part 1 around a suitably shaped handle 2 and the like. The bonding may also be temporary in a suitable manner, e.g. by means of threads, a bayonet or Luer lock or similar.

Especially convenient is the circular cross-section shown in Figure 2C or 2D, which makes "rolling" application possible to the surface of the dirty part of the area around the eye. During such application, the functional part 1 gradually applies pressure and relaxes around the perimeter, by which it facilitates adhesion of impurities. This mode of application also increases the area of the functional part 1 used and its undesirable friction against the cleaned area of the eye is reduced. The applicator end 1A of the functional part 1 is conveniently rounded, e.g. hemispherical, and the cross-section of the functional part 1 is conveniently tapered from the handle 2 to the applicator end 1A, as schematically shown in Figure 3. The length of the functional part 1 approximately conveniently between 3 mm and 30 mm, most conveniently between 5 and 20 mm. The transverse dimension (e.g. maximum diameter) of the functional part 1 is conveniently between 2 and 10 mm, best between 4 and 8 mm. The length of handle 2 may be from approximately 10 mm to 100 mm, conveniently between 30 and 65 mm.

The material of functional part 1 must be adequately soft, conveniently deformable with the use of minimum force, in order to allow expulsion of part of the fluid from the pores without damaging or irritating the cleaned area of the eye. For this reason, the volume of the pores should be more than 20%, and conveniently 30% of the hydrated volume of part 1. The porosity should be between 15% and 98%, conveniently between 25% and 90% of volume.

Also important is the size of the pores, whose average diameter should be between approximately 0.1 to 500 microns, conveniently from approx. 1 to 200 microns. It is convenient for the size of the pores not to be uniform, but have a certain distribution of diameters, corresponding, for instance, to Gaussian distribution. It is also possible to distribute the pores from inside outwards toward the surface, preferentially in such a way that the size of the pores also increases toward the surface of the functional part 1.

Also important is the wettability of the pore surfaces, so that a positive capillary pressure is preferentially created in the pores. To this purpose, it is convenient to select polymers, whose surface has a wetting angle of less than 90° to the physiologically unobjectionable fluid contained in the pores at room temperature and conveniently less than 75°. Hydrogels are mainly convenient, i.e. polymers insoluble in water, but that absorb water (that swell in the presence of water) up to a certain volume. Hydrogels may be covalently or physically cross-linked polymers of various chemical composition. Convenient for the purpose of the invention are mainly low to medium hydrophilic hydrogels with balanced water content from 20% weight to 60% weight, preferentially between 30% and 50% weight, which are tolerant also to fine fabrics and capable of surface absorption of impurities of both hydrophilic and hydrophobic character. That are also capable of holding water fluid in their pores, which may be expelled by relatively small mechanical pressure, which may be controlled by the size and hydrophilic character of the pores. For application, the porous polymer functional part 1 should be at least partially moistened with a physiologically unobjectionable fluid.

The main condition for selection of polymers for the functional part 1 is its health certification, mainly that is does not contain toxic or irritant substances or cannot emit such substances during storage and usage. Experts may design many polymers and copolymers that fulfil the functional conditions of the invention. So, for instance, polyurethanes and polyureas, including their resultant copolymers with polyols can be used. Another preferred class of polymers comprises vinyl polymers and copolymers, e.g. polymers and copolymers of polyvinyl alcohol or vinyl acetate. A further preferred class comprises polymers and copolymers of acrylates and methacrylates, such as polymeric amides and esters of acrylate and methacrylate acids. An example may be the sparsely cross-linked copolymer 2-hydroxyethyl methacrylate (HEMA) and small quantity of bisethylene glycol dimethacrylate. Also convenient are polymers containing iogenous groups, such as carboxyls, sulfonamides, tertiary amines, etc. Some of the possible compositions shall be stated in the Examples. The fluid contained in the pores is a non-toxic and non-irritant fluid, conveniently a salt solution or water miscible organic non-toxic compounds. Such organic compounds are, for instance, compounds with two or more hydroxyl groups, such as, sugars, glycerine, 1,2 propylene glycol, triethylene glycol, etc. Other suitable compounds may be dimethyl sulfoxide or glycerol formal. Such compounds may be present in the solution up to an approximate concentration of 80% weight, but usually their presence in a smaller volume also suffices. Isotonic solutions are mainly convenient, for instance, suitable salts, such as NaCl. This solution can further contain suitable antiseptics or other active substances, usually in ophthalmic formulations, e.g. in so-called boric acid. It is important that they are non-irritant to the eye and are adequately stable during storage and use of the Device. It is suitable for application that the overall fluid volume present in the functional part be adequately smaller than its maximum retention capacity for the given fluid. The retention capacity of porous hydrogel is understood to be the overall content of fluid retained in the structure of the hydrogel and the fluid retained in fully-filled pores. If the fluid content would be too high, then the fluid would be expelled from the pores during application and could flow from around the eye, smear the make-up, etc. If it would be too low (e.g. substantially lower than the balanced water content in the hydrogel), then the surface of the functional part could be too rough and damage or irritate the surface of the eye, edges of the eyelid, etc. According to our experience, the stated fluid should at the moment of contact with the eye area be contained in a volume equivalent to 20% to 95% and conveniently 33% to 67% of maximum retention capacity of the functional part from porous polymer. The fluid content may be adjusted just before application (e.g. wring out of the excess fluid, or on the contrary, soaking of a dry device in fluid), but it is more convenient to store and supply the Device suitably packed with an optimum fluid volume.

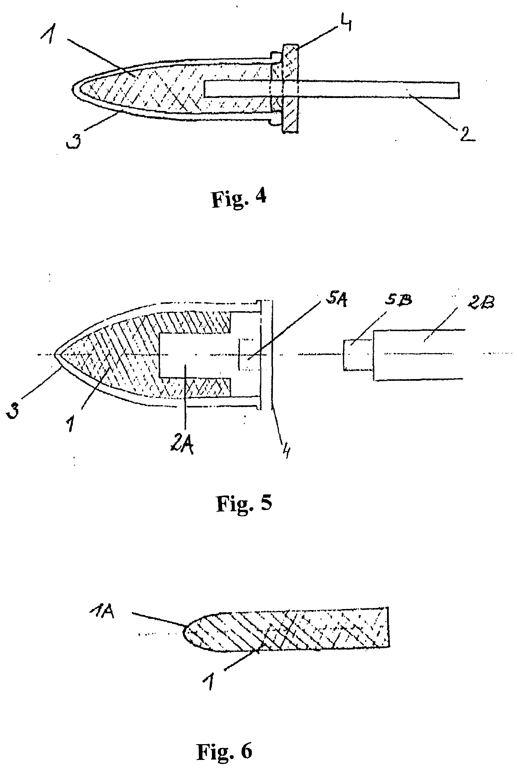

For this reason, apart from the functional part 1 and the handle 2, the device is conveniently has watertight packaging 3 that protects the functional part 1 from drying and contamination. The packaging 3 may contain more than one device, and these can be taken out gradually for usage. Such packaging may be, for instance, a bottle with a wide neck and screw cap, or plastic casing, etc. But individual packaging for each device is convenient as makes more comfortable usage possible and better guarantees a clean device. Such an individual packaging 3 may consist, for instance, of a plastic "blister" covered with removable film or packaging for the entire device from welded plastic film. Especially convenient is packaging 3 shaped from plastic material according to the shape of the functional part 1, as shown in Figure 4. The packaging is conveniently sealed with a cap 4 attached to the handle 2, and that eventually forms one integral part with it.

The entire device may be sealed in an impermeable packaging that protects it from contamination and retains the stated unobjectionable physiological fluid. In disposable design, this packaging could be made, for instance, of steam-proof plastic film, which can be torn to remove the device. Plastic film that is especially suitable for usage is, for instance, from polyethylene, polyethylene terephthalate (PET) or from metallized plastic film. The handle 2 may have an end adapted for easy tearing of the packaging. Another alternative is divided packaging that contains the device, preferentially two-piece packaging from suitable plastic material, such as, polypropylene (PP), where both parts are made by injection moulding and mutually connected by a watertight dismountable joint, such as a screw joint, Luer lock or bayonet lock. Such packaging may be used for one-time and repeated usage of the Device, eventually, also for its hygienic storage before disposal as waste.

Another convenient arrangement with the handle 2 consisting of two parts, which are the inner part 2A and outer part 2B, is schematically shown in Figure 5. The plastic packaging 3 has a watertight cap 4 and contains the functional part 1 and the inner part 2A of the device handle 2. The outer part 2B of the device handle 2 is not permanently connected to the inner part 2A, but is connected just before usage after opening the cap 4. The cap 4 and inner part 2A of the device handle 2 conveniently constitute a single component. After usage, the outer part 2B is disconnected from the inner part 2A, which is together with the connected functional part 1 and packaging 3 disposed of as waste, while the outer part 2B of the device handle 2 is kept for further usage. The inner part 2A and outer part 2B have a suitable system for quick coupling and release, such as the bayonet lock, Luer Lock, a lock that is usual in automatic pencils, etc., and which is schematically shown in Figure 5 as the nut part 5A and base part 5B.

A convenient method for production of the functional part 1 is casting of the liquid precursor in a mould under conditions suitable for creation of porous polymer. Such processes are well-known to experts in the plastics branch. The casting mould conveniently forms the packaging 3. The composition of the liquid precursor is conveniently selected so that the result of its hardening is non-toxic and non-irritant and it would not be necessary to extensively wash or clean it otherwise. This eliminates various production operations and reduces production costs. Moreover, the reduction of the number of operations also reduces the possibility of contamination. The convenient material for the functional part 1 is porous hydrogel. Creation of a spongy gel structure can be attained, for instance, by using a precursor that contains more water fluid than the created hydrogel can retain by balanced swelling. In such a case, so-called, "syneresis" occurs, whereby the polymer and fluid mutually exude each other resulting in the creation of a spongy structure. This effect is well-known to polymer chemists, who can find may concrete systems that fulfil this condition. An example of the creation of a porous hydrogel by means of a physical process may be, for instance, recurrent freezing and thawing of polyvinyl alcohol solutions, which by freezing and subsequent thawing create a fine spongy gel structure, whose consistency, porosity, etc. can be controlled, e.g. by the concentration of the polymer in the solution, by the temperature and number of cycles. Another example may be the co-polymerisation of HEMA with a suitable cross-linker in adequately diluted water solutions. The initiation of polymerisation using radical initiators is suitable, for instance, organic or inorganic peroxides or Azo-compounds. Initiators are decomposed by heat, UV radiation or reaction with suitable reducing compounds (so-called, redox initiation systems). Such water-soluble initiators whose reaction products are unobjectionable in terms of health are especially convenient and the products need not be removed carefully from the reaction. An example may be hydrogen peroxide or Ammonium persulfate.

The hardening of the liquid precursor is conveniently done in the presence of the handle 2 in a form, which is thus installed in the hydrogel functional part 1 without the need for additional operations. However, it is alternatively possible to create a hole or depression in the functional part 1 as a receptor for the handle 2, and bond the functional part 2 to this receptor using a suitable adhesive, e.g. cyanoacrylate glue.

The basic function of the device is removal of dirt from the eye area, as has been described already. However, the device may be used also for other hygienic purposes, for instance, cleaning of other body cavities, application or removal or repair of cosmetics devices, removal of excessive fluids, etc. The basic cleaning function of the device can be described as follows:
- the device is taken out of the packaging 3, the fluid content in it is eventually adjusted, and it is introduced to the eye area, such that the applicator end 1A of its functional part 1 touches the location of the dirt to be removed;
- the dirt upon contact sticks to the porous polymer without injuring or irritating the eye tissue;
- the device is removed from the eye area together with the dirt.

After removal from the eye area, it is possible to clean the device, wet it with fresh water fluid and re-use it. The advantages in terms of user hygiene, safety and comfort is, however, discarding of the device after first use.

### Examples

### Example 1

The polyethylene container with a volume of 1.5 ml with cap is used primarily as a mini test-tube or sample container for small samples (FISCHER SCIENTIFIC, Catalogue N° 2103.3502); it is used as a casting mould and as a watertight packaging 3 (see Figure 4 and Figure 5) for the hydrogel functional part 1. The mould is modified by cutting off the cap, which is then replaced by the polyethylene part that is used as an anchor for the inner part 2A, the cap 4 and the nut part 5A with bayonet lock according to Figure 5. The mould is filled with a 10% PVA and 0.8% NaCl water solution, closed from above with the stated polyethylene part and subjected to 3 cycles of freezing to - 20°C and thawing to + 20°C. The solution is prepared by dissolving PVA of molecular weight 80k Dalton and hydrolysis level above 99% in a isotonic solution of NaCl at a temperature above 95°C. By recurrent freezing and thawing, the polymer is exuded in insoluble form from the water and porous hydrogel with open pores is created, from which it is possible to easily wring excess water by application of small pressure. The product is stored in this form. Before use, the metallic exterior part 2B of handle 2 is attached to the cap 4 according to Figure 5; the handle has the nut part 5B with bayonet lock at its end, and the porous hydrogel functional part 1 is pulled from the packaging 3 and used to clean the eye area. After this, the handle 2 is disengaged and kept for further use, while the used functional part 1 is returned to the packaging 3 and discarded as waste.

### Example 2

The polyethylene mould from example 1 is partly filled with a de-aerated solution of 30% weight HEMA in a 1% ammonium sulphate water solution. HEMA contains 0.25 % weight of ethylene glycol dimethacrylate and 5 % weight triethylene glycol dimethacrylate. The filled mould is then closed with the rubber cap 4, which is attached to a polypropylene handle 2 according to Figure 4. The closed moulds are heated in a water bath to 80°C over a period of 3 hours, after which they are cooled and stored in a plastic container. By thermal polymerisation, the hydrogel sponge than constitutes the functional part 1 according to Figure 4, is created in the mould consisting of the packaging 3. Before use, the hydrogel functional part 1 is removed from the packaging 3 using the handle 2 and the sounded applicator end 1A of functional part 1 is used to clean the eye area. After cleaning, the functional part 1 including the handle 2 and cap 4 is returned to the packaging 3, which is disposed of as waste at an opportune moment.

### Example 3

The procedure from example 2 is repeated up to the end of the thermal polymerisation. After this, the functional part 1 with cap 4 and handle 2 from Figure 4 is removed from the mould, the cap 4 is removed and recycled. The functional part 1 with handle 2 are then washed in distilled water and soaked in a 30% glycerine water solution. After 24 hours, the excess solution is drained by spinning and the parts are dried in the air for 12 hours. The water partly evaporates from the functional part 1 and concentrated glycerine is retained in it, which serves as a softener and partly as a hydrogel preservative. The finished product is weld-sealed in polyethylene film. Before use, the film is torn, the product is taken out and used to clean the eye area either directly, or after moistening with an ophthalmic solution. Upon finding dust, foreign bodies, cosmetics residues or mascara particles in the made-up areas around the eyes, the device is taken out of the plastic bag according to the example and eventually also moistened with an ophthalmic solution. The stick is placed on the dirt lightly hydrogel part first and the dirt is removed by rotary motion. This prevents smearing of the eye make-up.

The same procedure can be applied to a case of an eyelash turned into the eye. The device for removal of dirt from the eye area is placed with hydrogel functional part on such eyelash and the lash is removed by rotary motion. We thus prevent further irritation of the eye.

Upon finding organic waste products (rheum) in the canthus (some people suffer from chronic creation of rheum - even several times a day), the rheum is comfortably removed by rotary motion of the Device moistened with an ophthalmic solution without smearing the make-up or irritating or infecting the eye.

When removing the rheum from animals, the procedure is the same, only the size of the functional hydrogel part is larger. The rotation of the functional part moistened with ophthalmic solution can also be used to soak the hair stuck around the eye, which also irritate the eye area.

## Claims

1. Device for removal of undesirable substances and items, such as metabolic waste products, soiling, dust and cosmetic residues from around the eyes, consisting of a functional part (1) and a handle (2), the handle (2) permanently bonded to or separable from the functional part (1); and a watertight packaging (3), in which the functional part (1) and optionally the handle (2) are sealed, **characterised in that** the functional part (1) consists of a porous hydrophilic polymer which is shaped for non-aggressive contact with the cleaned area of the eye and comprises in its porousness a non-toxic and non-irritant fluid.

2. The device according to claim 1, **characterized in that** the porous hydrophilic polymer of the functional part (1) has an average porosity of 20 to 98 % by volume, related to the total volume of the porous hydrophilic polymer.

3. The device according to any of the claims 1 to 2, **characterized in that** the porous hydrophilic polymer of the functional part (1) has an average pore size of 0,1 to 250 microns.

4. The device according to any of the claims 1 to 3, **characterized in that** the porous hydrophilic polymer of the functional part (1) is deformable porous hydrophilic polymer adapted for pressing out the absorbed non-toxic and non-irritant fluid by deformation of the functional part (1).

## Patentansprüche

1. Vorrichtung zum Entfernen von unerwünschten Substanzen und Objekten wie Stoffwechselausscheidungsprodukten, Verschmutzungen, Staub und Kosmetikrückständen aus der Augenumgebung, bestehend aus einem funktionalen Teil (1) und einem Griff (2), wobei der Griff (2) dauerhaft mit dem funktionalen Teil (1) verbunden oder von diesem trennbar ist; und eine wasserdichte Verpackung (3), in der das funktionale Teil (1) und optional der Griff (2) dichtend eingeschlossen sind,
**dadurch gekennzeichnet, dass** das funktionale Teil (1) aus einem porösen hydrophilen Polymer besteht, das zur nicht angreifenden Berührung mit dem gereinigten Bereich des Auges geformt ist und in seiner Porosität ein nicht toxisches und nicht reizendes Fluid umfasst.

2. Vorrichtung nach Anspruch 1, **dadurch gekennzeichnet, dass** das poröse hydrophile Polymer des funktionalen Teils (1) eine durchschnittliche Porosität von 20 bis 98 Volumen-% aufweist, in Bezug auf das Gesamtvolumen des porösen hydrophilen Polymers.

3. Vorrichtung nach einem der Ansprüche 1 bis 2, **dadurch gekennzeichnet, dass** das poröse hydrophile Polymer des funktionalen Teils (1) eine durchschnittliche Porengröße von 0,1 bis 250 Mikrometern aufweist.

4. Vorrichtung nach einem der Ansprüche 1 bis 3, **dadurch gekennzeichnet, dass** das poröse hydrophile Polymer des funktionalen Teils (1) ein deformierbares poröses hydrophiles Polymer ist, das zum Herausdrücken des absorbierten, nicht toxischen und nicht reizenden Fluids durch Deformation des funktionalen Teils (1) ausgelegt ist.

## Revendications

1. Dispositif pour le retrait de substances et éléments indésirables, tels que des produits de déchets métaboliques, des salissures, de la poussière et des résidus cosmétiques provenant du contour des yeux, consistant en une partie fonctionnelle (1) et un manche (2), le manche (2) étant lié de façon permanente à la partie fonctionnelle (1) ou étant séparable de celle-ci ; et un emballage (3) étanche à l'eau, dans lequel la partie fonctionnelle (1) et facultativement le manche (2) sont scellés, **caractérisé par le fait que** la partie fonctionnelle (1) consiste en un polymère hydrophile poreux qui est façonné en vue d'un contact non agressif avec la zone nettoyée de l'œil et comprend, dans sa porosité, un fluide non toxique et non irritant.

2. Dispositif selon la revendication 1, **caractérisé par le fait que** le polymère hydrophile poreux de la partie fonctionnelle (1) a une porosité moyenne de 20 à 98 % en volume, par rapport au volume total du polymère hydrophile poreux.

3. Dispositif selon l'une quelconque des revendications 1 et 2, **caractérisé par le fait que** le polymère hydrophile poreux de la partie fonctionnelle (1) a une taille de pore moyenne de 0,1 à 250 microns.

4. Dispositif selon l'une quelconque des revendications 1 à 3, **caractérisé par le fait que** le polymère hydrophile poreux de la partie fonctionnelle (1) est un polymère hydrophile poreux déformable apte à exprimer le fluide non toxique et non irritant absorbé par déformation de la partie fonctionnelle (1).
